# EUROPEAN PATENT SPECIFICATION

(11) **EP 4 612 703 B1**
(45) Date of publication and mention of the grant of the patent: **11.03.2026**
(21) Application number: 23790343.0
(22) Date of filing: 18.10.2023
(51) Int. Cl.: G16H 30/20, H04L 65/80, A61B 5/00, A61B 8/00, G16H 80/00, G16H 40/67, H04L 65/752, H04L 65/75, A61B 6/00

(54) **MAINTAINING A TELECONFERENCING CONNECTION WITH A MINIMUM RESOLUTION AND/OR FRAMERATE**
AUFRECHTERHALTUNG EINER TELEKONFERENZVERBINDUNG MIT EINER MINIMALEN AUFLÖSUNG UND/ODER EINER BILDRATE
MAINTIEN D'UNE CONNEXION DE TÉLÉCONFÉRENCE AVEC UNE RÉSOLUTION ET/OU UNE FRÉQUENCE DE TRAME MINIMALE

(30) Priority: 03.11.2022 EP 22205255
(43) Date of publication of application: 10.09.2025
(73) Proprietor: Koninklijke Philips N.V., 5656 AG Eindhoven (NL)
(72) Inventor: VAN GENUGTEN, Lenneke, 5656 AG Eindhoven (NL); VAN EE, Raymond, 5656 AG Eindhoven (NL); JELFS, Sam Martin, 5656 AG Eindhoven (NL); MEULENDIJKS, Paul, 5656 AG Eindhoven (NL); ANAND, Shreya, 5656 AG Eindhoven (NL); CHAKRABARTI, Biswaroop, 5656 AG Eindhoven (NL)
(74) Representative: Philips Intellectual Property & Standards
(86) International application number: PCT/EP2023/078906
(87) International publication number: WO 2024/094424

(56) References cited:
- CN-A- 113 891 036
- US-A1- 2009 012 821
- US-A1- 2014 357 993
- US-B2- 11 372 913
- YOO S K ET AL: "Design of a PC-based multimedia telemedicine system for brain function teleconsultation", INTERNATIONAL JOURNAL OF MEDICAL INFORMATICS, ELSEVIER SCIENTIFIC PUBLISHERS, SHANNON, IR, vol. 61, no. 2-3, 1 May 2001 (2001-05-01), pages 217 - 227, XP004235203, ISSN: 1386-5056, DOI: 10.1016/S1386-5056(01)00143-5

## Description

### FIELD OF THE INVENTION

The invention relates to teleconferencing in medicine.

### BACKGROUND OF THE INVENTION

Teleconferencing is a cost-effective means of allowing remote persons to participate in a medical examination, training exercise, or discussion.

United States patent application publication US2010318380A1 discloses a controller configured to allow flexible control of information distribution for telemedical assistance being provided via a telemedicine workplace for a treatment carried out in a treatment room. The controller is configured for receiving medical information from a medical modality or storage system for medical information, forwarding the received medical information to a display of the treatment room, controlling the forwarding of information to the treatment room, forwarding the received medical information to a display of the telemedicine workplace, and controlling the forwarding of the received medical information to the telemedicine workplace.

United States patent application publication US20140357993A1 discloses video stream of live medical images is generated at a local site having a medical image scanner. A live video stream is transmitted to at least one remote site via a network, which may include wired or wireless Internet connections. Network conditions are monitored during a network session and predictions are made on a predicted bit rate for transmission. The compression parameters for the live video stream are selected based on the predicted bit rate.

The journal publication Yoo et. al., "Design of a PC-based multimedia telemedicine system for brain function teleconsultation," International Journal of Medical Informatics, Volume 61, Issues 2-3, 2001, Pages 217-227, ISSN 1386-5056, https://doi.org/10.1016/S1386-5056(01)00143-5, discloses that during time-critical brain surgery, the detection of developing cerebral ischemia is particularly important because early therapeutic intervention may reduce the mortality of the patient. The purpose of this system is to provide an efficient means of remote teleconsultation for the early detection of ischemia, particularly when subspecialists are unavailable. The hardware and software design architecture for the multimedia brain function teleconsultation system including the dedicated brain function monitoring system is described. In order to comprehensively support remote teleconsultation, multi-media resources needed for ischemia interpretation were included: EEG signals, CSA, CD-CSA, radiological images, surgical microscope video images and video conferencing.

### SUMMARY OF THE INVENTION

The invention provides for a medical system, a computer program, and a method as defined in the independent claims. Embodiments are given in the dependent claims.

In medical teleconferencing it can be difficult to provide the proper amount of information to a remote user. If there are multiple video streams, limited bandwidth may cause the degradation or corruption of useful information. Embodiments may provide for an improved means of teleconferencing by using a medical system that is configured to set a minimum resolution and/or minimum framerate for one of the multiple video streams. In order to maintain his minimum resolution and/or framerate the other video streams (chosen from the multiple video streams and excluding the one of the multiple videos streams) have their framerate reduced and/or their resolution reduced and/or are halted or paused to provide sufficient network bandwidth.

In one aspect the invention provides for a medical system that comprises a memory that stores machine-executable instructions. The medical system further comprises a camera system that is configured for providing at least one subject video stream. The camera system may therefore comprise one or more video cameras. In some examples there may be one or more video cameras for imaging a subject. In other examples there may be, alternatively or in addition to, one or more cameras for imaging a presenter.

The medical system further comprises a medical video source that is configured to provide at least one medical video stream. The medical video source may take different forms in different examples. In one example the medical video source is a medical imaging system such as an ultrasound system or a magnetic resonance imaging system. The at least one medical video stream would then comprise medical images acquired by this medical imaging system. In other examples the medical video source could be a user interface where a presenter is reviewing or discussing medical images such as radiological or tomographic medical images. In yet other examples, the medical video source could be streamed from a repository or storage space which stores medical images or videos. In yet other examples the medical video source could be an external video feed of a remote presenter.

The medical system further comprises a network interface that is configured to form a network connection with one or more remote communication devices. A remote communication device as used herein is a device which is configured for displaying video information that it receives via the network connection. The medical system further comprises a computational system. Execution of the machine-executable instructions causes the computational system to form a teleconferencing connection with the one or more remote communication devices via the network connection. A teleconferencing connection as used herein encompasses a bidirectional connection via a network connection that enables two or more persons to hold a virtual discussion or meeting.

The teleconferencing connection comprises or is configured for providing multiple video streams. The multiple video streams comprise the at least one subject video stream and the at least one medical video stream. Execution of the machine-executable instructions further causes the computational system to determine a bandwidth of the network connection. The bandwidth is a measure of how much information or video data can be transmitted and/or received via the network connection. Execution of the machine-executable instructions further causes the computational system to receive a minimum resolution and/or a minimum frame rate for one of the multiple video streams. Depending upon the content of the one of the multiple video streams, it may be advantageous to specify a minimum resolution and/or a minimum frame rate. For example, if certain data is being looked at in a radiological image or the medical video source shows an image of a subject, the minimum resolution and/or the minimum frame rate may be necessary for something to be observable in the one of the multiple video streams.

Execution of the machine-executable instructions further causes the computational system to reduce a frame rate and/or resolution and/or halt transmission of another of the one or more video streams to ensure that the transmission of the multiple video streams does not exceed the bandwidth when enforcing the minimum resolution and/or minimum frame rate of the one of the multiple video streams. In this feature the other of the multiple video streams may be stopped and/or have their data transmission reduced in the form of a reduced resolution and/or frame rate in order to ensure that the one of the multiple video streams satisfies the specified condition. This may be beneficial because it may enable complicated medical data to be transmitted and disseminated to multiple users when there is a limit to the available bandwidth. A situation like this may be particularly advantageous because it is possible that there may be a large number of remote communication devices that are connecting via the network interface. This may be a way of ensuring that the minimum resolution and/or the minimum frame rate for one of the multiple video streams is maintained.

It should also be noted that the minimum resolution and the minimum frame rate for the one of the multiple video streams may change as a function of time depending upon various actions that are being performed.

In embodiments the medical system further comprises a medical imaging system. The medical video source comprises the medical imaging system. Execution of the machine-executable instructions further causes the computational system to control the medical imaging system to acquire measurement data. The measurement data as used herein encompasses the raw data acquired by the medical imaging system that is then used to reconstruct an image. Examples of these would include acoustic measurements by an ultrasound system, the k-space data in a magnetic resonance imaging system, or the profile data acquired by a computed tomography system. Execution of the machine-executable instructions further causes the computational system to reconstruct the medical image data from the measurement data. This embodiment may be beneficial because the live medical image data may be provided by the medical system to multiple individuals. For example, the medical image data may provide the video for the one of the multiple video streams which should have the minimum resolution and/or the minimum frame rate. This may be advantageous because it can ensure that the quality of the medical image data as it is presented in a raw form or via a user interface may be useful to remote users.

In another embodiment the at least one medical video stream comprises a rendering of a control user interface of the medical imaging system. This embodiment may be advantageous because it may provide for a means of presenting information from the user interface in a means which is then intelligible or able to be understood by remote users. This for example may be useful in discussing the repair of a medical imaging system, it may also be useful in a variety of other situations, for example, when a group of individuals are discussing the operation of the medical system, looking at live medical image data that is being acquired by the medical imaging system, or in reviewing data and discussing it.

In another embodiment the medical imaging system is a magnetic resonance imaging system.

In another embodiment the medical imaging system is a computed tomography system.

In another embodiment the medical imaging system is an ultrasound imaging system.

In another embodiment the medical imaging system is a positron emission tomography system.

In another embodiment the medical imaging system is a single-photon emission tomography system.

In another embodiment the medical imaging system is a digital X-ray system.

In another embodiment the medical imaging system is a digital fluoroscopy system.

In another embodiment the medical imaging system is an image-guided therapy device. Examples of image-guided therapy devices may include for example, an image-guided gamma knife that is guided with magnetic resonance imaging or a computed tomography system. Another example would be a LINAC that is guided with a magnetic resonance imaging system.

In another embodiment the medical imaging system is a magnetic resonance imaging-guided high-intensity focused ultrasound device.

In another embodiment the medical imaging system is a magnetic resonance imaging-guided radiotherapy device.

In another embodiment the medical imaging system is a functional near infrared spectroscopy system.

In another embodiment the medical imaging system is a computed tomography-guided radiotherapy device.

In embodiments execution of the machine-executable instructions further causes the computational system to determine a machine state of the medical imaging system during acquisition of the measurement data. Execution of the machine-executable instructions further causes the computational system to modify the minimum resolution and/or the minimum frame rate based on the machine state. This embodiment may be beneficial because as the medical imaging system is used, the minimum resolution and/or the minimum frame rate can be modified such that information about the medical imaging system can be accurately transmitted.

In another embodiment the medical video source comprises a rendering of a DICOM viewer user interface. This embodiment may be beneficial because it may enable the sharing of data on a DICOM viewer user interface to be shared with more individuals during a teleconference effectively. The medical video source further comprises a rendering of a user interface.

In another embodiment the medical video source further comprises a medical image database. For example, the video streams may be provided by retrieving historical or archived data from the medical image database.

In another embodiment the camera system is configured to image a subject. The memory further stores a body tracking module configured for providing body movement data descriptive of body movement of the subject in at least one subject video stream. Execution of the machine-executable instructions further causes the computational system to determine the body movement data by inputting the at least one subject video stream into the body tracking module. Execution of the machine-executable instructions further causes the computational system to modify the minimum resolution and/or minimum frame rate for one of the multiple video streams by applying a predetermined criterion to the body movement data. A body tracking module as used herein, is a software module that may be used to identify anatomical landmarks from an image or video of a subject. Various analytical techniques and neural network-based techniques are known for providing body movement data.

In another embodiment the body movement data comprises hand action data. The minimum resolution and/or the minimum frame rate is modified by applying the predetermined criterion to the hand action. For example, a minimum frame rate resolution for a region around the subject's hands may be used. Also, by modifying the hand action a particular action or activity of the subject may be noted. This may for example, provide a means of automatically adjusting the minimum resolution and/or the minimum frame rate when a physician or other healthcare provider is performing a particular task.

In another embodiment the body movement data comprises eye gaze location data. For example, by looking at a subject in an image the body movement data may record where a subject is looking. Likewise, the subject may also wear specialized eyewear which measures the eye gaze location data. The minimum resolution and/or the minimum frame rate is modified by applying the predetermined criterion to the eye gaze location data. For example, if one is presenting the data in the teleconference, the minimum resolution and/or the minimum frame rate can be set so that the information which the subject is looking at is also presented in a higher resolution or smoother with a higher frame rate to the participants who are at a remote location.

In another embodiment execution of the machine-executable instructions further causes the computational system to receive device-specific configuration data from the one or more remote communication devices. Execution of the machine-executable instructions further causes the computational system to adjust the minimum resolution and/or the minimum frame rate using the device-specific configuration data. This may be useful because it may provide for a means to a remote user for adjusting the video feeds being sent to this remote location. This for example, may be useful in enforcing better standards or quality of the video as well as enabling the reduction of bandwidth when it is not desired by the remote user.

In another embodiment the device-specific configuration data is received by applying configuration rules to device-specific user metadata. For example, there may be data which designates a user's position in the organization or contains specific data about the video needs of the user. This may provide for a means of better tailoring the minimum resolution and/or the minimum frame rate for a particular user.

In another embodiment one or more of the remote communication devices is a computer.

In another embodiment the one or more of the remote communication devices is a smartphone.

In another embodiment the one or more of the remote communication devices is a mobile telecommunications device.

In another embodiment the one or more of the remote communication devices is a DICOM user interface.

In another embodiment the one or more of the remote communication devices is a tablet computer.

In another embodiment the device-specific configuration data is user-generated configuration data received from a user interface of the one or more remote communication devices. For example, a user may manually enter the user-generated configuration data.

In another embodiment execution of the machine-executable instructions further causes the computational system to apply a subject data filter to the remote subject data from the multiple video streams. This for example may be useful to reduce a bandwidth of the multiple video streams. For example, if there is textual data or other information about a particular subject which is contained in the at least one medical video stream, a software module can be used to blur out or obscure or paint over this information. Causing this data to be removed may result in a reduction in the size of the multiple video streams.

The subject data filter may, for example, have an Optical Character Recognition (OCR) module which is able to identify or recognize textual data in the multiple video streams. A filter can be used to specify which information may be removed. In this case, a software module can then be used to paint over or black out the subject data which is detected. The encoding requirements for a blocked out or painted over region will be less than if there is more complex text data that is presented.

In another aspect the invention provides for a computer program that comprises machine-executable instructions for execution by a computational system that is configured for controlling a medical system. For example, the machine-executable instructions may be stored or embodied on a non-transitory storage medium. The medical system comprises a camera system that is configured for providing at least one subject video stream. The medical system further comprises a medical video source that is configured to provide at least one medical video stream. The medical video system further comprises a network interface that is configured to form a network connection with one or more remote communication devices.

Execution of the machine-executable instructions causes the computational system to form a teleconferencing connection with the one or more remote communication devices via the network connection. The teleconferencing connection comprises or is configured to provide multiple video streams. The multiple video streams comprise at least one subject video stream and the at least one medical video stream. Execution of the machine-executable instructions further causes the computational system to determine a bandwidth of the network connection. Execution of the machine-executable instructions further causes the computational system to receive a minimum resolution and/or the minimum frame rate for one of the multiple video streams.

Execution of the machine-executable instructions further causes the computational system to reduce a frame rate and/or resolution and/or halt transmission of another or at least one of the other of the one or more multiple video streams to ensure that the transmission of the multiple video streams does not exceed the bandwidth when enforcing the minimum resolution and/or the minimum frame rate of the one of the multiple video streams.

In another aspect the invention provides for a method of operating the medical system. The medical system comprises a camera system configured for providing at least one subject video stream. The medical system further comprises a medical video source configured to provide at least one medical video stream. The medical system further comprises a network interface configured to form a network connection with one or more remote communication devices.

The method comprises forming a teleconferencing connection with one or more remote communication devices via the network connection. The teleconferencing connection is configured for providing multiple video streams. The multiple video streams comprise the at least one subject video stream and the at least one medical video stream. The method further comprises determining a bandwidth of the network connection. The method further comprises receiving a minimum resolution and/or the minimum frame rate for one of the multiple video streams. The method further comprises reducing a frame rate and/or resolution and/or halt transmission of another of the one or more multiple video streams to ensure that the transmission of the multiple video streams does not exceed the bandwidth when enforcing the minimum resolution and/or minimum frame rate of the one of the multiple video streams.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A `user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A `hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Measurement data is defined herein as being recorded measurements made by a medical imaging system descriptive of a subject. The measurement data may be reconstructed into medical image data.

### BRIEF DESCRIPTION OF THE DRAWINGS

In the following preferred embodiments of the invention will be described, by way of example only, and with reference to the drawings in which:
Fig. 1 illustrates an example of a medical system;
Fig. 2 shows a flow chart which illustrates a method of using the medical system of Fig. 1;
Fig. 3 illustrates an example of a medical system;
Fig. 4 shows a flow chart which illustrates a method of using the medical system of Fig. 2; and
Fig. 5 illustrates an example of configuring multiple video streams.

### DESCRIPTION OF EMBODIMENTS

Like numbered elements in these figures are either equivalent elements or perform the same function. Elements which have been discussed previously will not necessarily be discussed in later figures if the function is equivalent.

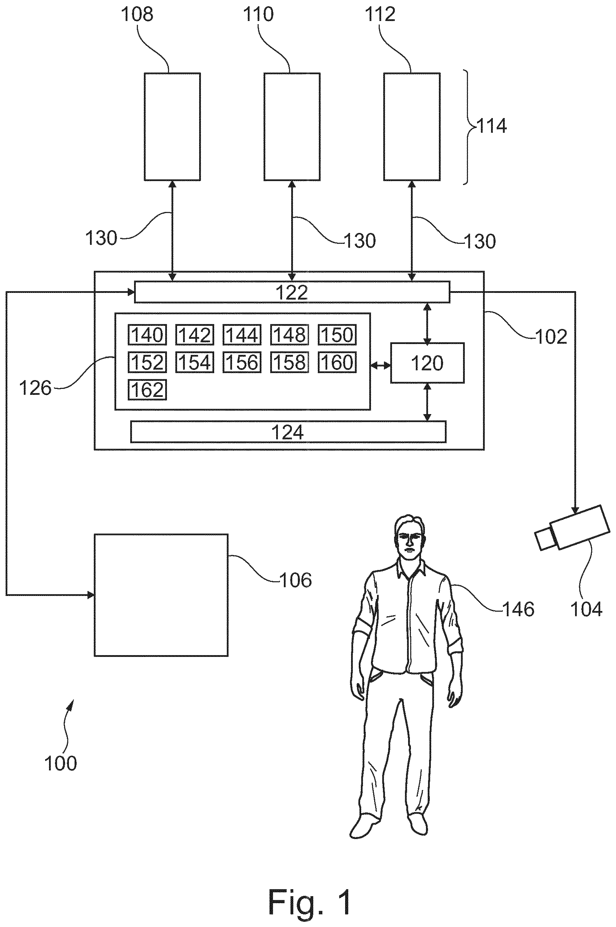

Fig. 1 illustrates an example of a medical system 100. The medical system is shown as comprising a computer 102. The medical system 100 is also shown as comprising a camera system 104 and a medical video source 106. Also shown is a smartphone 108, a tablet computer 110 and a remote computer 112. These are all examples of remote communication devices 114 that could form a teleconferencing connection 130 with the computer 102. The computer 102 is shown as comprising a computational system 120. The computational system 120 may represent one or more computing or computational cores at one or more locations. The computational system 120 is shown as being in communication with a hardware or network interface 122. The hardware or network interface 122 is used for forming the teleconferencing connection 130 with the remote communication devices 114. In this example, the hardware interface 122 is also used to form the connection with the medical video source 106 and the camera system 104.

The computational system 120 is further shown as being optionally connected to a user interface 124. The user interface 124 may for example provide a means for an operator to interact with and control the medical system 100. The computational system 120 is further shown as being in connection with a memory 126. The memory 126 is intended to represent various types of memories which may be accessible to the computational system 120. In some examples the memory 126 is a non-transitory storage medium.

The memory 126 is shown as containing machine-executable instructions 140. The machine-executable instructions 140 enable the computational system 120 to perform various tasks such as controlling other components of the medical system 100. The machine-executable instructions 140 also enable the computational system 120 to perform various computational and video processing tasks. The memory 126 is further shown as containing a medical video stream 142 that has been received from the medical video source 106 and a subject video stream 144 that has been received from the camera system 104. In this example, the camera system 104 is imaging a subject 146. The subject video stream 144 would then contain a video stream descriptive of the subject 146.

The medical video stream 142 and the subject video stream 144 are then combined into multiple video streams 148. The multiple video streams 148 are then transmitted as part of the teleconferencing connection 130. The memory 126 is further shown as containing a bandwidth measurement 150. This may be a bandwidth of the teleconferencing connection 130 to each of the remote communication devices 114 or a best bandwidth measurement. The memory 126 is further shown as containing a minimum resolution and/or the minimum frame rate 152. The memory 126 is then shown as containing a selection of one of the multiple video streams 154 for which the minimum resolution and/or the minimum frame rate 152 is required. The memory 126 further shows the video stream control commands 156 which are used to adjust the contents of the medical video stream 142 and/or the subject video stream 144 such that when the one of the multiple video streams 154 has the minimum resolution and/or the minimum frame rate 152 the bandwidth 150 is met.

The execution of the video stream control commands 156 could for example cause the computational system 120 to reduce the frame rate and/or the resolution of streams in the multiple video streams 148 which are not the one of the multiple video streams 154.

The memory 126 is further shown as containing an optional body tracking module 158. The body tracking module 158 takes as input images of the subject 146 and outputs body movement data 160. This may be used to optionally adjust the minimum resolution and/or the minimum frame rate 152 as well as change a selection of which of the video streams is the one of the multiple video streams 154. For example, the actions of the subject 146 or an observation of where the subject 146 is touching or pointing or looking may be used to provide this modification.

The memory 126 is also further shown as containing an optional subject data filter 162. The optional subject data filter 162 is able to remove subject data from the medical video stream 142 and/or the subject video stream 144. For example, the subject data filter 162 may have an OCR filter, which is used to recognize specific subject data which may then be erased or blocked out in the multiple video streams 148. This may for example be useful in reducing the bandwidth of individual video streams in the multiple video streams 148.

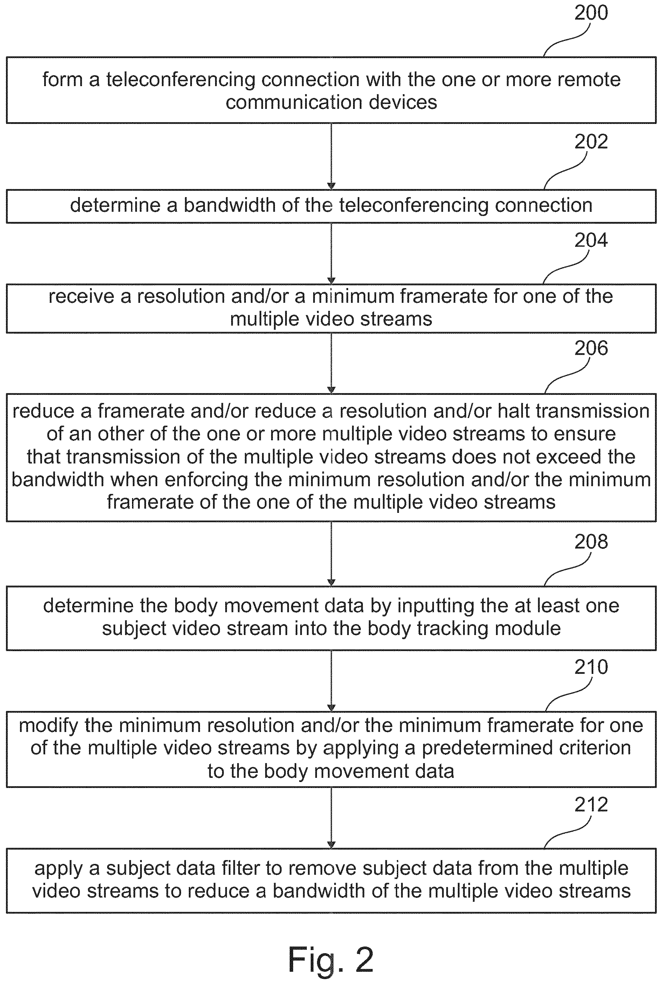

Fig. 2 shows a figure which illustrates a method of operating the medical system 100 of Fig. 1. First, in step 200, a teleconferencing connection 130 is formed with the one or more remote communication devices using the network interface 122. Next, in step 202, a bandwidth 150 of the teleconferencing connection 130 is determined. Then, in step 204, a minimum resolution and/or a minimum frame rate 152 is received for the multiple video streams 148. Then, in step 206, a reduction in the frame rate and/or a reduction in resolution and/or a halt transmission of another of the one or more video streams is performed to ensure that the transmission of the multiple video streams does not exceed the bandwidth when enforcing the minimum resolution and/or the minimum frame rate of the one of the multiple video streams. Steps 208, 210, and 212 are optionally performed. In step 208 the body movement data 160 is determined by inputting the at least one subject video stream into the body tracking module 158.

Next, in step 210, the minimum resolution and/or the minimum frame rate for one of the multiple video streams is modified by applying a predetermined criterion to the body movement data 160. Finally, in step 212, the subject data filter 162 is applied to the multiple video streams 148 to remove subject data from the multiple video streams to reduce a bandwidth of the multiple video streams.

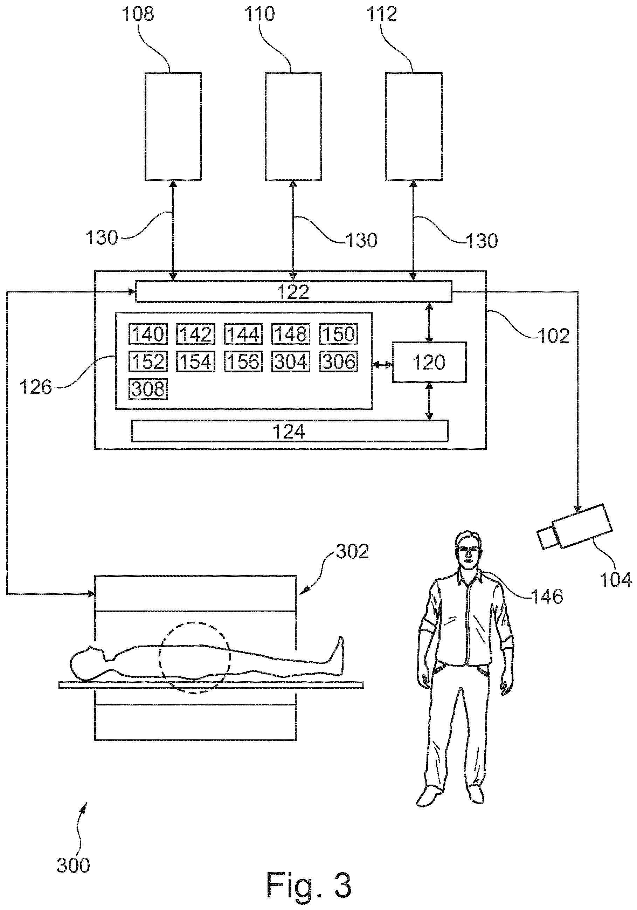

Fig. 3 illustrates a further example of a medical system 300. The medical system 300 in Fig. 3 is similar to the medical system 100 in Fig. 1. In this case the medical video source is a medical imaging system 302. Depending upon the imaging modality, the medical imaging system 302 could be able to provide a video stream itself. In some cases, the medical video stream 142 will be a video stream of a user interface for operating the medical imaging system 302 or examining images that were just acquired using the medical imaging system 302.

The memory 126 is further shown as containing measurement data 304. The measurement data 304 was acquired by the medical imaging system 302. The memory 126 is further shown as containing medical image data 306 that was reconstructed from the measurement data 304. The medical imaging system 302 is intended to represent medical imaging systems in general. The medical imaging system could for example be a magnetic resonance imaging system, a computed tomography system, an ultrasound imaging system, a positron emission tomography system, a single photon emission tomography system, a digital X-ray system, a digital fluoroscopy system, an image guided therapy device, a magnetic resonance imaging guided high intensity focused ultrasound device, a magnetic resonance imaging guided radiotherapy device, a functional near infrared spectroscopy system, or a computed tomography guided radiotherapy device.

In the example illustrated in Fig. 3 the medical imaging system 302 may have various internal machine states, such as when various controls or foot pedals are operated or when an X-ray tube is activated or if a radiofrequency system is in the process of acquiring k-space data for a magnetic resonance image. The memory 126 is shown as containing a machine state of the medical imaging system 308. This can be used for example, with a set of predetermined conditions or criterion to provide commands or instructions to modify the minimum resolution and/or the minimum frame rate 152. In this case, as the medical imaging system 302 passes through various phases or states of operation, the requirements of the multiple video streams 148 changes accordingly.

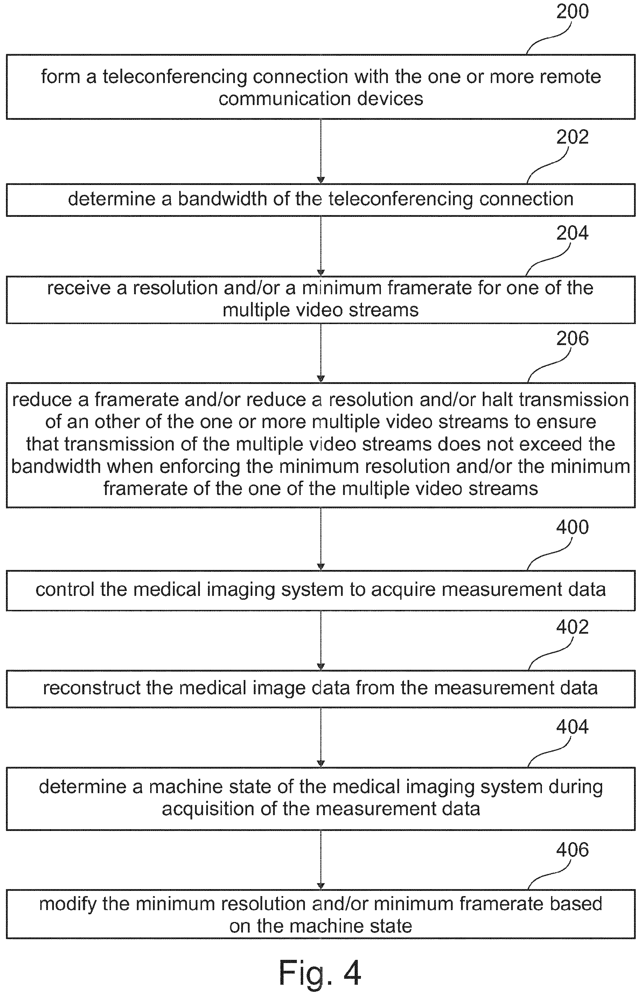

Fig. 4 shows a flowchart which illustrates a means of operating the medical system 300 of Fig. 3. In this flowchart steps 200, 202, 204, and 206 are as illustrated and explained in Fig. 2. After step 206 is performed step 400 is performed. In step 400 the medical imaging system 302 is controlled to acquire the measurement data 304. Next, in step 402, the medical image data 306 is reconstructed from the measurement data 304. Then, in step 404, the machine state 308 of the medical imaging system 302 is determined during acquisition of the measurement data 304. Finally, in step 406, the minimum resolution and/or the minimum frame rate are modified based on the machine state 308. It should be noted that step 404 may be performed during steps 400 and 402 also.

It is also noted that the embodiments depicted in Figs. 3 and 4 may be combined with the example illustrated in Figs. 1 and 2.

Physicians, clinicians & technicians, have a complex job that consist of many tasks. While preparing or using Image Guided Therapy (IGT), Magnetic Resonance Imaging (MRI), or Ultra Sound (US) technology, they may need guidance, appreciate a peer looking over their shoulder, or may be teaching at the same time.

It may be important that the teleconferencing software supports the specific procedural goals (e.g., that it precisely & timely shows what the distant party needs to see). However, the circumstances for the transfer of visual, audio, video and possibly haptic information to an external audience are not always optimal. For example, the connections may be slow because the bandwidth is insufficient. It may then be beneficial to not overburden the medical system with unnecessary information or features.

It may be beneficial if a medical system with a teleconferencing tool provides the right information to both parties. Too little information could lead to clinical problems (e.g., imprecisions); too much information to connection problems.

Examples may have the benefit of optimizing the communication needs and find the best configuration giving the context.

Examples may provide for a medical system that has teleconferencing settings would fit for a particular situation (e.g., based on bandwidth, image quality (resolution) needs, clinician characteristics, patient characteristics, privacy):
- Determine goals/ needs of (next part of) session
- Determine contextual factors
- Determine optimal settings to achieve goal(s) in this context
- Send out settings
- Feedback loop

Examples may also comprise an algorithm to combine hospital/medical/device information, parties involved and the goal of the session to form a recommendation of which information needs to be communicated and which information may be left out.

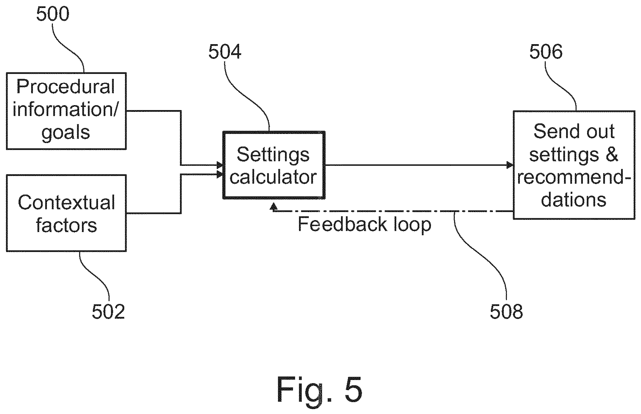

Fig. 5 illustrates an example of setting the configuration of the multiple video streams 148. Box 500 represents various procedural and information goals which may be used during a particular teleconference. Box 502 indicates a variety of contextual factors. These contextual factors 502 may for example be on various details about a procedure or operation performed in the vicinity of the teleconference or medical system 100, 300. Both of these information sources 500, 502 are input into a settings calculator 504, which provides various settings 506 for configuring the teleconferencing connection 130 to maintain the bandwidth. Various local operators may have certain requests or may emphasize the various video streams of the multiple video streams 148. A feedback loop 508 provides a means of modifying this.

In some examples, this may include the determination of procedural information to determine goals/ needs of a session. In this step, it is determined what the goals is (goals are) for this session. The main question is: What is the clinical task for this session? More specific questions are e.g. Who are working Together? What are their needs? How important is quality? E.g., is resolution or latency more important?

To gather this information as unobtrusive as possible, it may possibly be derived from hospital information. This includes but is not limited to the following information:
- Patient diagnosis, procedure(s) planned; details of procedure like duration, complexity, equipment needed, etc. This can be obtained from the patient medical records and room and equipment planning.
- E.g., for IGT, this can be done from 'procedure cards', if they used / selected beforehand: certain combinations of applications are going to be important to show together, procedure cards can provide this information in advance.
- Interaction task/ goal for this session. This involves the sub questions: who are working together? And what are their goals? E.g., is this a peer to peer session, professor-student, something else? Is it for guidance, teaching, proctoring or consultation, etc.? This information can also be derived from hospital information systems.

For example, staff registries to determine job titles, which inform about seniority of the people involved (and their potential learning needs).

Calendar information on the procedure & attendants
o What are sending 'needs' from user 1 and receiving 'needs' from user 2 (,3,4, etc.)? Need to know their current knowledge & skill level. Look at their role, derive information from planning system staff records, etc.

Furthermore, for the remote user (i.e., staff-in-training), they fill in / choose their information needs before a session. Typically, the remote user does have some time/motivation to interact with the system as opposed to staff
- Understanding of where the action/ relevant information is happening, e.g. from Physician hands information, from movement in video or catheter input ( (real time input).

Contextual factors may also be used in some examples to determine possibilities and limitations of the interaction:
In this step, information is automatically collected, aimed at understanding the context(s) in which the system has to operate.
- Information may be collected at sender & receivers' side
   - Static system information, such as monitor size
   - Variable system information such as connection information, incl speed, consistency, reliability, bandwidth; constant feedback/ keep updating this information. This may be assessed continuously or very regularly.
- Information on what is happening in the room of interest, e.g.
   - Input from a remote viewer or system:
   - Hand action
   - Eye gaze: what physician is looking at, is probably most relevant (with some exceptions of when a remote proctor/student/SalesRep, wants to check other sources such as settings / vital signs / historical images etc. when the physician is doing his/her thing)
   - Moving images / last updated images / sources (e.g., anglio runs)
   - Foot pedal input (when x-ray is taken it's typically about the live image then)
   - Certain actions require zooming in (e.g., measuring lesions or vessels), so a higher resolution is needed.

Examples may also comprise a settings calculator (which could for example be implemented using the machine executable instructions 140): Determine optimal settings to achieve goal(s) in this context

This system may possibly work in 2 steps: information prioritization followed by contextual analysis.

Step 1: prioritization of information. Based on session goals (and momentary goals), the system determines which information needs to be prioritized.
- This includes priorities for user 1 AND 2 (and etc.). It determines what needs to be send & received from each, based on the information from the first element (determine goals).
- Patient safety is always at the top of the priority list.
   - It may take into account severity of consequences when failing to provide the right information at the right moment. This may include using scientific information & hospital data to gather that information

Step 2: these goals are weighed against contextual factors and normative (experience and or population) data. This could include determining what are technical possibilities in this context. For equal priority, equal weighting factors are given.
- System that weighs these goals vs context; Could be rule based, given priority list
- Some underlying requirements:
   - When applications/images linked to devices (e.g., IVUS images) change, we know they are moving the device, and it's probably useful to show both views. If there are multiple cameras, it would be helpful to provide a synthesized view/outline from the point of view of the operator to the remote user
   - When there is anything happening with a device (e.g., catheter, stent placement, ect.) is it important to show the combination of hand movements + device. For example, for IGT, when there is specific question or novelty in using the device (e.g., device sales representative support / proctoring on a specific technique).
      - Hand movements with the device are always relevant in combination with the live image (if available) for certain procedure steps (e.g., access, stent placements). (Though this view could be much smaller than the clinical image).

      ∘ When to show the general OR view: This view is never essential but gives a good context and is greatly appreciated in remote training or guidance by support staff. Clinicians are also positive, but see it as a nice to have for giving remote support / 2nd opinions are provided. It's also a standard if you want to broadcast it as a (live) case in lectures or at conferences. It's about: the layout of the lab, who is involved in which location, but also just gives 'a human factor' to it. Some key moments:
         - During the introduction of the case (before sharing specific information)
         - Changes in setup (e.g., c-arm movements)
         - 'In between moments', it's a good fall-back view.
   - The clinical staff of the operating side may be bothered as little as possible. For the remote user, it's quite acceptable for the remote user to have to zoom/click/select different views as long as it avoids the clinical staff having to be involved. Thus, the system could decide to e.g., simply increase / decrease resolution or framerate based on their input; the remote user can then `zoom in' so it can increase the resolution; if desired. Or select their own camera sources.
- It is then decided which information is broadcasted to which receiver

Output: send out teleconferencing settings

In this phase, the system updates the settings that determine which information is relayed (and which not) with which speed, quality
- This includes settings with respect to different information sources: text, video, audio (e.g., detect phase; explanation vs modelling)
- Based on the clinical task and modality used, usage settings can also be recommended. For example, settings for an MR course will be different from setting for a IGT course.
   - Each modality (CT, MR, US) have different settings for different tasks (clinical condition of the patient) . For e.g.: different views in terms of x-ray, lateral oblique, etc.

Based on the clinical condition to be imaged and modality ordered, the settings can be recommended.
- a look-up table is used to adapt to what receiving party do/ see/ request.
- Can we mention a few examples of IGT settings here?
   - call settings one could change in current design: e.g., what camera input is shared, who is muted etc.)
   - Call settings in potential future design, e.g., what viewport is shared / what grid or layout is it presented in, what is the resolution it's shared in

In some examples a feedback loop may be used to improve success rates.

In order to improve user satisfaction and the success rate of the system, there are various ways to learn about the performance of the system.
- At any time, users (both senders & receivers) can override output settings.
- If users 'ask' for additional information, this is stored as well (e.g., turns on full room camera while system chose to not display this). If specific extra information is regularly requested the system can automatically add it to the default for those procedures.
- Users can manually change weightings of priorities and feed this information back into the system for future usage.
- A post-event survey that assesses (all) users' satisfaction; and specifically, dissatisfaction with data streamed, e.g., what were they missing?
- For complex procedures when one or more of the active participants are working remotely, it may be beneficial to explicitly read out steps from a checklist among the participants (similar to airplane pilots' SOP). The system would employ voice recognition and NLP to validate completeness

The medical system may take this kind of feedback into account, relates it to the specific goals & context of that setting, and improves the rules/ algorithm for these instances. The settings recommender can be a multi-class classification, where a Convolution Neural Network is used for classification. The procedural information and contextual factors will be the two inputs to the network and a set of settings would be the various buckets for classification. In a scenario, where the recommended setting is modified/altered by the user, the modified setting is added to the list as feedback for further training.

This basic medical system can be enriched with one or more of the following examples:
- In determining goal: continuously keep updating interaction goal as the relevance of sound, video or medical image may change over time. This includes the setting of subgoals (subsequent over time) based on the primary goals determined in the first step.
- technical measures/aspects that are being measured live/real time, such as monitor size, etc.
- Users can add, remove & adapt 'basic rules' in the calculator, e.g., on which views which preferably go together, which clinician to focus on, setting an alarm when connection quality drops below a certain threshold, etc.

Examples of the medical system may be applied in many settings:
- For remote conferencing, education, counselling, proctoring, guidance, teaching, etc.
- For clinical support (as described here) but also e.g., technical support, so systems like ROCC, and users like biomeds, RSEs, etc.
- For multiple clinical modalities: Precision Diagnostics (PD), Computed Tomography (CT), Image Guided Therapy (IGT), Magnetic Resonance (MR), and etc.
   - the above examples are often IGT specific. A similar system can have similar benefits also for MR/CT but would be generally less complex.
   - For example PD modalities (CT/DXR/MR), generally don't do procedures so there is no point in a 'live case' broadcast if you will. Technologists can also 'share a screen' rather straightforwardly when they want a radiologist to have a look at the image quality for example. There is a trade off between speed/IQ here too but it's less urgent.
- And perhaps also in regular meetings between colleagues.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical system
- 102: computer
- 104: camera system
- 106: medical video source
- 108: smart phone
- 110: tablet computer
- 112: remote computer
- 114: remote communication device
- 120: computational system
- 122: hardware / network interface
- 124: user interface
- 126: memory
- 130: teleconferencing connection
- 140: machine executable instructions
- 142: medical video stream
- 144: subject video stream
- 146: subject
- 148: multiple video streams
- 150: bandwidth measurement
- 152: minimum resolution and/or minimum framerate
- 154: one of the multiple video streams
- 156: video stream control commands
- 158: body tracking module
- 160: body movement data
- 162: subject data filter
- 200: form a teleconferencing connection with the one or more remote communication devices via a network connection
- 202: determine a bandwidth of the network connection
- 204: receive a minimum resolution and/or a minimum framerate for one of the multiple video streams
- 206: reduce a framerate and/or resolution and/or halt transmission of an other of the one or more multiple video streams to ensure that transmission of the multiple video streams does not exceed the bandwidth when enforcing the minimum resolution and/or the minimum framerate of the one of the multiple video streams
- 300: medical system
- 302: medical imaging system
- 304: measurement data
- 306: medical image data
- 308: machine state of medical imaging system
- 400: control the medical imaging system to acquire measurement data
- 402: reconstruct the medical image data from the measurement data
- 404: determine a machine state of the medical imaging system during acquisition of the measurement data
- 406: modifying the minimum resolution and/or a minimum framerate based on the machine state
- 500: Procedural information / goals
- 502: Contextual factors
- 504: Settings calculator
- 506: Send out settings and recommendations
- 508: Feedback loop

## Claims

1. A medical system (100, 300) comprising:
- a memory (126) storing machine executable instructions (140);
- a camera system (104) configured for providing at least one subject video stream (144);
- a medical video source (106) configured to provide at least one medical video stream (142);
- a medical imaging system (302), wherein the medical video source comprises the medical imaging system;
- a network interface (122) configured to form a network connection with one or more remote communication devices;
- a computational system (120), wherein execution of the machine executable instructions causes the computational system to:
- form (200) a teleconferencing connection (130) with the one or more remote communication devices via the network connection, wherein the teleconferencing connection is configured for providing multiple video streams (148), wherein the multiple video streams comprise the at least one subject video stream and the at least one medical video stream;
- determine (202) a bandwidth (150) of the network connection;
- receive (204) a minimum resolution and/or a minimum framerate (152) for one of the multiple video streams;
- reduce (206) a framerate and/or resolution and/or halt transmission of an other of the multiple video streams to ensure that transmission of the multiple video streams does not exceed the bandwidth when enforcing the minimum resolution and/or the minimum framerate of the one of the multiple video streams;
- control (400) the medical imaging system to acquire measurement data (304);
- reconstruct (402) medical image data from the measurement data;
- determine (404) a machine state (308) of the medical imaging system during acquisition of the measurement data; and
- modify (406) the minimum resolution and/or a minimum framerate based on the machine state.

2. The medical system of claim 1, wherein the at least one medical video stream comprises a rendering of a control user interface of the medical imaging system.

3. The medical system of claim 1 or 2, wherein the medical imaging system is any one of the following: a magnetic resonance imaging system, a computed tomography system, an ultrasound imaging system, a positron emission tomography system, a single photon emission tomography system, a digital X-ray system, a digital fluoroscopy system, an image guided therapy device, a magnetic resonance imaging guided high intensity focused ultrasound device, a magnetic resonance imaging guided radiotherapy device, a functional near infrared spectroscopy system, and a computed tomography guided radiotherapy device.

4. The medical system of any one of the preceding claims, wherein the medical video source comprises any one of the following: a rendering of a DICOM viewer user interface, a rendering of a user interface, a medical image database, and combinations thereof.

5. The medical system of any one of the preceding claims, wherein the camera system is configured to image a subject (146), wherein the memory further stores a body tracking (158) module configured for providing body movement data (160) descriptive of body movement of the subject in the at least one subject video stream, wherein execution of the machine executable instructions further causes the computational system to:
- determine (208) the body movement data by inputting the at least one subject video stream into the body tracking module; and
- modify (210) the minimum resolution and/or the minimum framerate for one of the multiple video streams by applying a predetermined criterion to the body movement data.

6. The medical system of claim 5, wherein anyone of the following:
- the body movement data comprises hand action data, wherein the minimum resolution and/or the minimum framerate is modified by applying the predetermined criterion to the hand action;
- the body movement data comprises eye gaze location data, wherein minimum resolution and/or the minimum framerate is modified by applying the predetermined criterion to the eye gaze location data; and
- combinations thereof.

7. The medical system of any one of the preceding claims, wherein execution of the machine executable instructions further causes the computational system to:
- receive device specific configuration data from the one or more remote communication devices; and
- adjust the minimum resolution and/or a minimum framerate using the device specific configuration data.

8. The medical system of claim 7, wherein the device specific configuration data is received by applying configuration rules to device specific user metadata.

9. The medical system of any one of the preceding claims, wherein the one or more remote communication devices is any one of the following: a computer, a smart phone, a mobile telecommunications device, a DICOM user interface, and a tablet computer.

10. The medical system of any one of claims 7, 8, or 9, wherein the device specific configuration data is user generated configuration data received from a user interface of the one or more remote communication devices.

11. The medical system of any one of the preceding claims, wherein execution of the machine executable instructions further causes the computational system to apply (212) a subject data filter (162).

12. The medical system of any one of the preceding claims, wherein the machine state is a state of operation.

13. The medical system of any one of the preceding claims, wherein the machine state is any one of the following:
- an operation of a control of the medical imaging system;
- an operation of a foot pedal of the medical imaging system;
- an activation of an X-ray tube, the medical system comprising an X-ray system; and
- an activation of a radio frequency system to acquire k-space data, the medical system comprising a magnetic resonance imaging system.

14. A computer program product comprising machine executable instructions (140) for execution by a computational system (120) configured for controlling a medical system (100, 300), wherein the medical system comprises a camera system (104) configured for providing at least one subject video stream (142), wherein the medical system further comprises a medical video source (106) configured to provide at least one medical video stream (142), wherein the medical system further comprises a medical imaging system (302), wherein the medical video source comprises the medical imaging system, wherein the medical system further comprises a network interface (122) configured to form a network connection with one or more remote communication devices, wherein execution of the machine executable instructions causes the computational system to:
- form (200) a teleconferencing connection (130) with the one or more remote communication devices via the network connection, wherein the teleconferencing connection is configured for providing multiple video streams (148), wherein the multiple video streams comprise the at least one subject video stream and the at least one medical video stream;
- determine (202) a bandwidth (150) of the network connection;
- receive (204) a minimum resolution and/or a minimum framerate for one of the multiple video streams;
- reduce (206) a framerate and/or resolution and/or halt transmission of an other of the multiple video streams to ensure that transmission of the multiple video streams does not exceed the bandwidth when enforcing the minimum resolution and/or the minimum framerate of the one of the multiple video streams;
- control (400) the medical imaging system to acquire measurement data (304);
- reconstruct (402) medical image data from the measurement data;
- determine (404) a machine state (308) of the medical imaging system during acquisition of the measurement data; and
- modify (406) the minimum resolution and/or a minimum framerate based on the machine state.

15. A method of operating a medical system (100, 300), wherein the medical system comprises a camera system (104) configured for providing at least one subject video stream (144), wherein the medical system further comprises a medical video source (106) configured to provide at least one medical video stream (142), wherein the medical system further comprises a medical imaging system (302), wherein the medical video source comprises the medical imaging system, wherein the medical system further comprises a network interface (122) configured to form a network connection with one or more remote communication devices,
wherein the method comprises
- forming (200) a teleconferencing connection (130) with the one or more remote communication devices via the network connection, wherein the teleconferencing connection is configured for providing multiple video streams (148), wherein the multiple video streams comprise the at least one subject video stream and the at least one medical video stream;
- determining (202) a bandwidth (150) of the network connection;
- receiving (204) a minimum resolution and/or a minimum framerate (152) for one of the multiple video streams;
- reducing (206) a framerate and/or resolution and/or halt transmission of an other of the multiple video streams to ensure that transmission of the multiple video streams does not exceed the bandwidth when enforcing the minimum resolution and/or the minimum framerate of the one of the multiple video streams;
- controlling (400) the medical imaging system to acquire measurement data (304);
- reconstructing (402) medical image data from the measurement data;
- determining (404) a machine state (308) of the medical imaging system during acquisition of the measurement data; and
- modifying (406) the minimum resolution and/or a minimum framerate based on the machine state.

## Patentansprüche

1. Medizinisches System (100, 300), umfassend:
- einen Speicher (126), der maschinenausführbare Anweisungen (140) speichert;
- ein Kamerasystem (104), das zum Bereitstellen mindestens eines Subjekt-Videostreams (144) konfiguriert ist;
- eine medizinische Videoquelle (106), die konfiguriert ist, um mindestens einen medizinischen Videostream (142) bereitzustellen;
- ein medizinisches Bildgebungssystem (302), wobei die medizinische Videoquelle das medizinische Bildgebungssystem umfasst;
- eine Netzwerkschnittstelle (122), die konfiguriert ist, um eine Netzwerkverbindung mit einer oder mehreren entfernten Kommunikationsvorrichtungen zu bilden;
- ein Rechensystem (120), wobei die Ausführung der maschinenausführbaren Anweisungen das Rechensystem veranlasst:
- eine Telekonferenzverbindung (130) mit der einen oder den mehreren entfernten Kommunikationsvorrichtungen über die Netzwerkverbindung zu bilden (200), wobei die Telekonferenzverbindung zum Bereitstellen mehrerer Videostreams (148) konfiguriert ist, wobei die mehreren Videostreams den mindestens einen Subjekt-Videostream und den mindestens einen medizinischen Videostream umfassen;
- eine Bandbreite (150) der Netzwerkverbindung zu bestimmen (202);
- eine Mindestauflösung und/oder eine Mindesteinzelbildrate (152) für einen der mehreren Videostreams zu empfangen (204);
- eine Einzelbildrate und/oder Auflösung zu verringern (206) und/oder eine Übertragung eines anderen der mehreren Videostreams anzuhalten, um zu gewährleisten, dass eine Übertragung der mehreren Videostreams beim Erzwingen die Mindestauflösung und/oder der Mindesteinzelbildrate des einen der mehreren Videostreams nicht die Bandbreite übersteigt;
- das medizinische Bildgebungssystems zu steuern (400), um Messungsdaten (304) zu erfassen;
- medizinische Bilddaten aus den Messungsdaten zu rekonstruieren (402);
- einen Maschinenzustand (308) des medizinischen Bildgebungssystems während einer Erfassung der Messungsdaten zu bestimmen (404); und
- die Mindestauflösung und/oder eine Mindesteinzelbildrate basierend auf dem Maschinenzustand zu modifizieren (406).

2. Medizinisches System nach Anspruch 1, wobei der mindestens eine medizinische Videostream ein Rendering einer Steuerungsbenutzeroberfläche des medizinischen Bildgebungssystems umfasst.

3. Medizinisches System nach Anspruch 1 oder 2, wobei das medizinische Bildgebungssystem eines von Folgendem ist: ein Magnetresonanzbildgebungssystem, ein Computertomografiesystem, ein Ultraschallbildgebungssystem, ein Positronenemissionstomografiesystem, ein Einzelphotonenemissionstomografiesystem, ein digitales Röntgensystem, ein digitales Durchleuchtungssystem, eine bildgeführte Therapievorrichtung, eine magnetresonanzbildgebungsgeführte fokussierte Ultraschallvorrichtung hoher Intensität, eine magnetresonanzbildgebungsgeführte Strahlentherapievorrichtung, ein funktionelles Nahinfrarot-Spektroskopiesystem und eine computertomografiegeführte Strahlentherapievorrichtung.

4. Medizinisches System nach einem der vorstehenden Ansprüche, wobei die medizinische Videoquelle eines von Folgendem umfasst: ein Rendering einer DICOM-Viewer-Benutzeroberfläche, ein Rendering einer Benutzeroberfläche, eine medizinische Bilddatenbank und Kombinationen davon.

5. Medizinisches System nach einem der vorstehenden Ansprüche, wobei das Kamerasystem konfiguriert ist, um ein Subjekt (146) abzubilden, wobei der Speicher weiter ein Körperverfolgungsmodul (158) speichert, das zum Bereitstellen von Körperbewegungsdaten (160) konfiguriert ist, die eine Körperbewegung des Subjekts in dem mindestens einen Subjekt-Videostream beschreiben, wobei Ausführen der maschinenausführbaren Anweisungen das Rechensystem weiter veranlasst:
- die Körperbewegungsdaten durch Eingaben des mindestens einen Patienten-Videostreams in das Körperverfolgungsmodul zu bestimmen (208); und
- die Mindestauflösung und/oder die Mindesteinzelbildrate für einen der mehreren Videostreams durch Anwenden eines vorbestimmten Kriteriums auf die Körperbewegungsdaten zu modifizieren (210).

6. Medizinisches System nach Anspruch 5, wobei eines von Folgendem gilt:
- die Körperbewegungsdaten umfassen Handaktionsdaten, wobei die Mindestauflösung und/oder die Mindesteinzelbildrate durch Anwenden des vorbestimmten Kriteriums auf die Handaktion modifiziert wird;
- die Körperbewegungsdaten umfassen Blickpositionsdaten, wobei die Mindestauflösung und/oder die Mindesteinzelbildrate durch Anwenden des vorbestimmten Kriteriums auf die Blickpositionsdaten modifiziert wird; und
- Kombinationen davon.

7. Medizinisches System nach einem der vorstehenden Ansprüche, wobei Ausführen der maschinenausführbaren Anweisungen das Rechensystem weiter veranlasst:
- vorrichtungsspezifische Konfigurationsdaten von der einen oder mehreren entfernten Kommunikationsvorrichtungen zu empfangen; und
- die Mindestauflösung und/oder eine Mindesteinzelbildrate unter Verwendung der vorrichtungsspezifischen Konfigurationsdaten anzupassen.

8. Medizinisches System nach Anspruch 7, wobei die vorrichtungsspezifischen Konfigurationsdaten durch Anwenden von Konfigurationsregeln auf vorrichtungsspezifische Benutzermetadaten empfangen werden.

9. Medizinisches System nach einem der vorstehenden Ansprüche, wobei die eine oder mehrere entfernte Kommunikationsvorrichtungen eine von Folgendem sind: ein Computer, ein Smartphone, eine mobile Telekommunikationsvorrichtung, eine DICOM-Benutzeroberfläche und ein Tablet-Computer.

10. Medizinisches System nach einem der Ansprüche 7, 8 oder 9, wobei die vorrichtungsspezifischen Konfigurationsdaten vom Benutzer erzeugte Konfigurationsdaten sind, die von einer Benutzeroberfläche der einen oder mehreren entfernten Kommunikationsvorrichtungen empfangen werden.

11. Medizinisches System nach einem der vorstehenden Ansprüche, wobei Ausführen der maschinenausführbaren Anweisungen das Rechensystem weiter veranlasst, einen Subjektdatenfilter (162) anzuwenden.

12. Medizinisches System nach einem der vorstehenden Ansprüche, wobei der Maschinenzustand ein Betriebszustand ist.

13. Medizinisches System nach einem der vorstehenden Ansprüche, wobei der Maschinenzustand einer von Folgendem ist:
- ein Betrieb einer Steuerung des medizinischen Bildgebungssystems;
- ein Betrieb eines Fußpedals des medizinischen Bildgebungssystems;
- eine Aktivierung einer Röntgenröhre, wobei das medizinische System ein Röntgensystem umfasst; und
- eine Aktivierung eines Hochfrequenzsystems, um k-Raum-Daten zu erfassen, wobei das medizinische System ein Magnetresonanzbildgebungssystem umfasst.

14. Computerprogrammprodukt, das maschinenausführbare Anweisungen (140) zur Ausführung durch ein Rechensystem (120), das zum Steuern eines medizinischen Systems (100, 300) konfiguriert ist, umfasst, wobei das medizinische System ein Kamerasystem (104) umfasst, das zum Bereitstellen mindestens eines Subjekt-Videostreams (142) konfiguriert ist, wobei das medizinische System weiter eine medizinische Videoquelle (106) umfasst, die konfiguriert ist, um mindestens einen medizinischen Videostream (142) bereitzustellen, wobei das medizinische System weiter ein medizinisches Bildgebungssystem (302) umfasst, wobei die medizinische Videoquelle das medizinische Bildgebungssystem umfasst, wobei das medizinische System weiter eine Netzwerkschnittstelle (122) umfasst, die konfiguriert ist, um eine Netzwerkverbindung mit einer oder mehreren entfernten Kommunikationsvorrichtungen zu bilden, wobei Ausführen der maschinenausführbaren Anweisungen das Rechensystem veranlasst:
- eine Telekonferenzverbindung (130) mit der einen oder den mehreren entfernten Kommunikationsvorrichtungen über die Netzwerkverbindung zu bilden (200), wobei die Telekonferenzverbindung zum Bereitstellen mehrerer Videostreams (148) konfiguriert ist, wobei die mehreren Videostreams den mindestens einen Subjekt-Videostream und den mindestens einen medizinischen Videostream umfassen;
- eine Bandbreite (150) der Netzwerkverbindung zu bestimmen (202);
- eine Mindestauflösung und/oder eine Mindesteinzelbildrate für einen der mehreren Videostreams zu empfangen (204);
- eine Einzelbildrate und/oder Auflösung zu verringern (206) und/oder eine Übertragung eines anderen der mehreren Videostreams anzuhalten, um zu gewährleisten, dass eine Übertragung der mehreren Videostreams beim Erzwingen die Mindestauflösung und/oder der Mindesteinzelbildrate des einen der mehreren Videostreams nicht die Bandbreite übersteigt;
- das medizinische Bildgebungssystems zu steuern (400), um Messungsdaten (304) zu erfassen;
- medizinische Bilddaten aus den Messungsdaten zu rekonstruieren (402);
- einen Maschinenzustand (308) des medizinischen Bildgebungssystems während einer Erfassung der Messungsdaten zu bestimmen (404); und
- die Mindestauflösung und/oder eine Mindesteinzelbildrate basierend auf dem Maschinenzustand zu modifizieren (406).

15. Verfahren zum Betreiben eines medizinischen Systems (100, 300) konfiguriert ist, umfasst, wobei das medizinische System ein Kamerasystem (104) umfasst, das zum Bereitstellen mindestens eines Subjekt-Videostreams (144) konfiguriert ist, wobei das medizinische System weiter eine medizinische Videoquelle (106) umfasst, die konfiguriert ist, um mindestens einen medizinischen Videostream (142) bereitzustellen, wobei das medizinische System weiter ein medizinisches Bildgebungssystem (302) umfasst, wobei die medizinische Videoquelle das medizinische Bildgebungssystem umfasst, wobei das medizinische System weiter eine Netzwerkschnittstelle (122) umfasst, die konfiguriert ist, um eine Netzwerkverbindung mit einer oder mehreren entfernten Kommunikationsvorrichtungen zu bilden, wobei das Verfahren umfasst
- Bilden (200) einer Telekonferenzverbindung (130) mit der einen oder den mehreren entfernten Kommunikationsvorrichtungen über die Netzwerkverbindung, wobei die Telekonferenzverbindung zum Bereitstellen mehrerer Videostreams (148) konfiguriert ist, wobei die mehreren Videostreams den mindestens einen Subjekt-Videostream und den mindestens einen medizinischen Videostream umfassen;
- Bestimmen (202) einer Bandbreite (150) der Netzwerkverbindung;
- Empfangen (204) einer Mindestauflösung und/oder einer Mindesteinzelbildrate (152) für einen der mehreren Videostreams;
- Verringern (206) einer Bildrate und/oder Auflösung und/oder Anhalten einer Übertragung eines anderen der mehreren Videostreams, um zu gewährleisten, dass eine Übertragung der mehreren Videostreams beim Erzwingen die Mindestauflösung und/oder der Mindesteinzelbildrate des einen der mehreren Videostreams nicht die Bandbreite übersteigt;
- Steuern (400) des medizinischen Bildgebungssystems, um Messungsdaten (304) zu erfassen;
- Rekonstruieren (402) medizinischer Bilddaten aus den Messungsdaten;
- Bestimmen (404) eines Maschinenzustandes (308) des medizinischen Bildgebungssystems während einer Erfassung der Messungsdaten; und
- Modifizieren (406) der Mindestauflösung und/oder einer Mindesteinzelbildrate basierend auf dem Maschinenzustand.

## Revendications

1. Système médical (100, 300) comprenant :
- une mémoire (126) stockant des instructions exécutables par machine (140) ;
- un système de caméras (104) configuré pour fournir au moins un flux vidéo sujet (144) ;
- une source vidéo médicale (106) configurée pour fournir au moins un flux vidéo médical (142) ;
- un système d'imagerie médicale (302), dans lequel la source vidéo médicale comprend le système d'imagerie médicale ;
- une interface réseau (122) configurée pour former une connexion réseau avec un ou plusieurs dispositifs de communication à distance ;
- un système informatique (120), dans lequel l'exécution des instructions exécutables par machine amène le système informatique à :
- la formation (200) d'une connexion de téléconférence (130) avec les un ou plusieurs dispositifs de communication à distance via la connexion réseau, dans laquelle la connexion de téléconférence est configurée pour fournir plusieurs flux vidéo (148), dans lesquels les flux vidéo multiples comprennent le au moins un flux vidéo sujet et le au moins un flux vidéo médical ;
- la détermination (202) d'une bande passante (150) de la connexion réseau ;
- la réception (204) d'une résolution minimale et/ou d'une fréquence d'images minimale (152) pour l'un parmi les flux vidéo multiples ;
- la réduction (206) d'une fréquence d'images et/ou d'une résolution et/ou l'interruption de la transmission d'un autre parmi les flux vidéo multiples afin de garantir que la transmission des flux vidéo multiples ne dépasse pas la bande passante lors de l'application de la résolution minimale et/ou de la fréquence d'images minimale de l'un parmi les flux vidéo multiples ;
- la commande (400) du système d'imagerie médicale pour acquérir des données de mesure (304) ;
- la reconstruction (402) de données d'image médicale à partir des données de mesure ;
- la détermination (404) d'un état machine (308) du système d'imagerie médicale pendant l'acquisition des données de mesure ; et
- la modification (406) de la résolution minimale et/ou d'une fréquence d'images minimale basée sur l'état machine.

2. Système médical selon la revendication 1, dans lequel le au moins un flux vidéo médical comprend un rendu d'une interface utilisateur de commande du système d'imagerie médicale.

3. Système médical selon la revendication 1 ou 2, dans lequel le système d'imagerie médicale est l'un des systèmes suivants : un système d'imagerie par résonance magnétique, un système de tomodensitométrie, un système d'imagerie par ultrasons, un système de tomographie par émission de positons, un système de tomographie d'émission monophotonique, un système de radiographie numérique, un système de fluoroscopie numérique, un dispositif de thérapie guidée par l'image, un dispositif d'ultrasons focalisés de haute intensité guidé par imagerie par résonance magnétique, un dispositif de radiothérapie guidée par imagerie par résonance magnétique, un système de spectroscopie fonctionnelle proche infrarouge et un dispositif de radiothérapie guidée par tomodensitométrie.

4. Système médical selon l'une quelconque des revendications précédentes, dans lequel la source vidéo médicale comprend l'un des éléments suivants : le rendu d'une interface utilisateur de visionneuse DICOM, le rendu d'une interface utilisateur, une base de données d'images médicales ou une combinaison de ceux-ci.

5. Système médical selon l'une quelconque des revendications précédentes, dans lequel le système de caméras est configuré pour imager un sujet (146), dans lequel la mémoire stocke en outre un module de suivi corporel (158) configuré pour fournir des données de mouvement corporel (160) descriptives d'un mouvement corporel du sujet dans le au moins un flux vidéo sujet, dans lequel l'exécution des instructions exécutables par machine amène en outre le système informatique à :
- déterminer (208) les données de mouvement corporel en saisissant le au moins un flux vidéo sujet dans le module de suivi corporel ; et
- modifier (210) la résolution minimale et/ou la fréquence d'images minimale pour l'un parmi les flux vidéo multiples en appliquant un critère prédéterminé aux données de mouvement corporel.

6. Système médical selon la revendication 5, dans lequel l'un quelconque des éléments suivants :
- les données de mouvement corporel comprennent des données d'action de la main, dans lequel la résolution minimale et/ou la fréquence d'images minimale est modifiée en appliquant le critère prédéterminé à l'action de la main ;
- les données de mouvement corporel comprennent des données de localisation du regard, dans lequel une résolution minimale et/ou la fréquence d'images minimale est modifiée en appliquant le critère prédéterminé aux données de localisation du regard ; et
- des combinaisons de ceux-ci.

7. Système médical selon l'une quelconque des revendications précédentes, dans lequel l'exécution des instructions exécutables par machine amène en outre le système informatique à :
- recevoir des données de configuration spécifiques au dispositif provenant d'un ou plusieurs dispositifs de communication à distance ; et
- ajuster la résolution minimale et/ou une fréquence d'images minimale à l'aide des données de configuration spécifiques au dispositif.

8. Système médical de la revendication 7, dans lequel les données de configuration spécifiques au dispositif sont reçues en appliquant des règles de configuration aux métadonnées utilisateur spécifiques au dispositif.

9. Système médical selon l'une quelconque des revendications précédentes, dans lequel les un ou plusieurs dispositifs de communication à distance sont l'un quelconque des éléments suivants : un ordinateur, un smartphone, un appareil de télécommunications mobiles, une interface utilisateur DICOM et une tablette.

10. Système médical selon l'une quelconque des revendications 7, 8 ou 9, dans lequel les données de configuration spécifiques au dispositif sont des données de configuration générées par l'utilisateur et reçues d'une interface utilisateur des un ou plusieurs dispositifs de communication à distance.

11. Système médical selon l'une quelconque des revendications précédentes, dans lequel l'exécution des instructions exécutables par machine amène en outre le système informatique à appliquer (212) un filtre de données sujet (162).

12. Système médical selon l'une quelconque des revendications précédentes, dans lequel l'état machine est un état de fonctionnement.

13. Système médical selon l'une quelconque des revendications précédentes, dans lequel l'état machine est l'un quelconque des éléments suivants :
- une opération de commande du système d'imagerie médicale ;
- une opération d'une pédale du système d'imagerie médicale ;
- une activation d'un tube à rayons X, le système médical comprenant un système à rayons X ; et
- une activation d'un système de radiofréquence pour acquérir des données de l'espace k, le système médical comprenant un système d'imagerie par résonance magnétique.

14. Programme informatique comprenant des instructions exécutables par machine (140) destinées à être exécutées par un système informatique (120) configuré pour commander un système médical (100, 300), dans lequel le système médical comprend un système de caméras (104) configuré pour fournir au moins un flux vidéo sujet (142), dans lequel le système médical comprend en outre une source vidéo médicale (106) configurée pour fournir au moins un flux vidéo médical (142), dans lequel le système médical comprend en outre un système d'imagerie médicale (302), dans lequel la source vidéo médicale comprend le système d'imagerie médicale, dans lequel le système médical comprend en outre une interface réseau (122) configurée pour former une connexion réseau avec un ou plusieurs dispositifs de communication à distance, dans lequel l'exécution des instructions exécutables par machine amène le système informatique à :
- former (200) une connexion de téléconférence (130) avec les un ou plusieurs dispositifs de communication à distance via la connexion réseau, dans laquelle la connexion de téléconférence est configurée pour fournir des flux vidéo multiples (148), dans lesquels les flux vidéo multiples comprennent le au moins un flux vidéo sujet et le au moins un flux vidéo médical ;
- déterminer (202) une bande passante (150) de la connexion réseau ;
- recevoir (204) une résolution minimale et/ou une fréquence d'images minimale pour l'un parmi les flux vidéo multiples ;
- réduire (206) une fréquence d'images et/ou une résolution et/ou interrompre la transmission d'un autre des flux vidéo multiples afin de garantir que la transmission des flux vidéo multiples ne dépasse pas la bande passante lors de l'application de la résolution minimale et/ou de la fréquence d'images minimale de l'un parmi les flux vidéo multiples ;
- commander (400) le système d'imagerie médicale pour acquérir des données de mesure (304) ;
- reconstruire (402) des données d'image médicale à partir des données de mesure ;
- déterminer (404) un état machine (308) du système d'imagerie médicale pendant l'acquisition des données de mesure ; et
- modifier (406) la résolution minimale et/ou une fréquence d'images minimale basée sur l'état machine.

15. Procédé de fonctionnement d'un système médical (100, 300), dans lequel le système médical comprend un système de caméras (104) configuré pour fournir au moins un flux vidéo sujet (144), dans lequel le système médical comprend en outre une source vidéo médicale (106) configurée pour fournir au moins un flux vidéo médical (142), dans lequel le système médical comprend en outre un système d'imagerie médicale (302), dans lequel la source vidéo médicale comprend le système d'imagerie médicale ; dans lequel le système médical comprend en outre une interface réseau (122) configurée pour former une connexion réseau avec un ou plusieurs dispositifs de communication à distance, dans lequel le procédé comprend
- la formation (200) d'une connexion de téléconférence (130) avec un ou plusieurs dispositifs de communication à distance via la connexion réseau, dans laquelle la connexion de téléconférence est configurée pour fournir des flux vidéo multiples (148), dans lesquels les flux vidéo multiples comprennent au moins un flux vidéo sujet et au moins un flux vidéo médical ;
- la détermination (202) d'une bande passante (150) de la connexion réseau ;
- la réception (204) d'une résolution minimale et/ou d'une fréquence d'images minimale (152) pour l'un parmi les flux vidéo multiples ;
- la réduction (206) d'une fréquence d'images et/ou d'une résolution et/ou l'interruption de la transmission d'un autre des flux vidéo multiples afin de garantir que la transmission des flux vidéo multiples ne dépasse pas la bande passante lors de l'application de la résolution minimale et/ou de la fréquence d'images minimale de l'un parmi les flux vidéo multiples ;
- la commande (400) du système d'imagerie médicale pour acquérir des données de mesure (304) ;
- la reconstruction (402) des données d'image médicale à partir des données de mesure ;
- la détermination (404) d'un état machine (308) du système d'imagerie médicale pendant l'acquisition des données de mesure ; et
- la modification (406) de la résolution minimale et/ou d'une fréquence d'images minimale basée sur l'état machine.
